# EUROPEAN PATENT SPECIFICATION

(11) **EP 2 956 547 B1**
(45) Date of publication and mention of the grant of the patent: **02.05.2018**
(21) Application number: 14707888.5
(22) Date of filing: 12.02.2014
(51) Int. Cl.: C12N 15/82, C07K 14/325

(54) **USE OF AXMI184 FOR THE CONTROL OF ROOTWORM INSECTS**
VERWENDUNG VON AXMI184 ZUR BEKÄMPFUNG VON MAISWURZELBOHRERN
UTILISATION D'AXMI184 POUR LA LUTTE CONTRE DES LARVES NUISIBLES AUX RACINES

(30) Priority: 13.02.2013 US 201361764246 P
(43) Date of publication of application: 23.12.2015
(73) Proprietor: Athenix Corp., Morrisville, NC 27560 (US)
(72) Inventor: MCNULTY, Brian, Chesterfield, MO 63017 (US); STAUFFER, Maria, Apex, NC 27523 (US)
(74) Representative: J A Kemp
(86) International application number: PCT/US2014/015997
(87) International publication number: WO 2014/126986

(56) References cited:
- WO-A1-99/57128
- WO-A2-03/075655
- WO-A2-2010/003065
- JUN FANG ET AL: "Characterization of chimeric Bacillus thuringiensis Vip3 toxins", APPLIED AND ENVIRONMENTAL MICROBIOLOGY, AMERICAN SOCIETY FOR MICROBIOLOGY, US, [Online] vol. 73, no. 3, 1 February 2007 (2007-02-01), pages 956-961, XP002670816, ISSN: 0099-2240, DOI: 10.1128/AEM.02079-06 Retrieved from the Internet: URL:http://aem.asm.org/content/73/3/956> [retrieved on 2006-11-01]

### SEQUENCE LISTING

<110> McNulty, Brian
   Stauffer, Maria
<120> USE OF AXMI184 FOR THE CONTROL OF ROOTWORM INSECTS
<130> APA136001
<150> 61/764,246
   <151> 2013-02-13
<160> 4
<170> PatentIn version 3.5
<210> 1
   <211> 2370
   <212> DNA
   <213> Bacillus thuringiensis
<400> 1
   atgaacatga acaagaataa tactaaatta agcgcaagag ctttaccaag ttttattgat 60
   tattttaatg gcatttatgg atttgccact ggcatcaaag atattatgaa catgattttt 120
   aaaacggata caggtggtga tctaacccta gacgaaattt taaagaatca gcagttacta 180
   aatgatattt ctggtaaatt ggatggggtg aatggaagct taaatgatct tatcgcacag 240
   ggaaacttaa atacagaatt atctaaggaa atattaaaaa ttgcaaatga acaaaatcaa 300
   gttttaaatg atgttaataa caaactcgat gcgataaata cgatgcttca tgtatatcta 360
   cctaaaatta cctctatgtt aagtgatgta atgaaaccaa attatgcgct aagtatgcaa 420
   atagaatacc taagtagaca attacaagaa atttcagata agctagatat tatcaacgta 480
   aatgtactta ttaactctac acttactgaa attacacctg cgtatcaatg gattaaatat 540
   gtgaacgaaa aatttgaaga attaactttt gctacagaaa ctactttaaa agtaaaaaat 600
   gatagcgctt ctgcagatat tcttgatgag ttaacggagt taactgaact tgcgaaaagt 660
   gtaacaaaaa atgatgtgga tggttttgaa ttttacctta atacattcca cgatgtaatg 720
   gtaggaaata atttattcgg gcgttcgact ttaaaaactg cttcggaatt aattgctaaa 780
   gaaaatgtga aaacaagtgg cagtgaggta ggaaatgttt ataatttctt agttgtatta 840
   acagctctac aagcaaaagc ttttcttact ttaacaacat gccgaaaatt attaggccta 900
   gcagatattg attatacatc tattatgaat gaacatttaa ataaggaaaa agaggaattt 960
   agagtaaaca tccttcctat actttctaat actttttcta atcctaatta tgcaaaagtt 1020
   aaaggaagtg atgaagatgc aaagatgatt gtggaagcta aaccaggaca tgcattggtt 1080
   gggtttgaaa ttagtaatga ttcaatgaca gtattaaaag tatatgaagc taagctaaaa 1140
   caaaattacc aagttgataa ggattcctta tcggaagtca tttatggtga tatggataaa 1200
   ttattgtgcc cagatcaatc tgaaaaaatt tattatacaa ataatatagt atttccaaat 1260
   gaatatgtaa ttactaaaat tgattttact aagaaaatga aaactttaag atatgaggta 1320
   acagctaatt cttatgattc ttctacagga gaaattgact taaataaaaa gaaagtagaa 1380
   tcaagtaaag cggagtatag gacgttaagt gctaataatg atggagtata tatgccgtta 1440
   ggtgtcatca gtgaaacatt tttgactcca attaatggat ttggcctcca agctgatgaa 1500
   aattcaagat taattacttt aacgtgtaaa tcatatttaa gggaactact actagcgaca 1560
   gacttaagca ataaagaaac taaattgatt gtcccgccaa atagttttat tagcaatatt 1620
   gtagagaatg ggtccataga agagggccac ttagagcctt ggaaagcaaa taataagaac 1680
   gcttatgtag atcatacagg cggagtgaat ggtactaaag ctctatatgt tcatgaggat 1740
   gggggggttt cacaatttat gggagataaa ttaaaaccga aaactgagta tgtaattcaa 1800
   tatactgtta aaggaaaacc ttctattcat ttaaaagatg aaaatactgg atatattctt 1860
   tatgaagata caaataatga tttagaagat ttccaaacta taactaaaag gttcacaaca 1920
   ggaactgatt taatgagagt gtatttaatt ttaaaaagtc aaagtggtca cgaagcttgg 1980
   ggagataact ttacaatttt ggaaattaag cctgcggagg ctttagtaag cccagaattg 2040
   attaatccga attcttggat tacaactcaa ggggctagca tttcaggaga taaacttttt 2100
   attagcttgg ggacaaatgg gacctttaga caaaatcttt cattaaacag ttattcaact 2160
   tatagtataa gctttactgc atcgggacca tttaatgtga cggtaagaaa ttctagggaa 2220
   gtattatatg aacgaaacaa ccttatgtct tcaactagtc atatttctgg ggaattcaaa 2280
   actgaatcca ataataccgg attatatgta gaactttccc gtcgttctgg tggtgctggt 2340
   catatatcat ttgaaaacat ttctattaaa 2370
<210> 2
   <211> 2370
   <212> DNA
   <213> Artificial sequence
<220>
   <223> synthetic gene encoding AXMI184 (optAxmi184)
<400> 2
   atgaacatga acaagaacaa caccaagctc tccgcgcgcg cgctgccctc cttcatcgac      60
   tacttcaatg gcatctatgg cttcgccacc ggcatcaagg acatcatgaa catgatcttc     120
   aagacagaca ctggaggaga tctcaccttg gatgagatcc tcaagaacca gcagctgctg     180
   aatgacatct cagggaagct ggatggcgtc aatggaagcc tcaatgatct cattgctcaa     240
   ggaaacctca acacagagct ctccaaggag atcctcaaga tcgccaatga gcagaaccag     300
   gtgctgaatg atgtcaacaa caagctggat gccatcaaca ccatgctgca tgtttatctt     360
   ccaaagatca cctcaatgct ctctgatgtg atgaagccaa actatgctct ctccatgcaa     420
   attgagtacc tctcaaggca gctgcaagag atctccgaca agctggacat catcaatgtc     480
   aatgtgctga tcaacagcac cttgacagag atcacgccgg cctaccaatg gatcaaatat     540
   gtcaatgaga agtttgagga gctcaccttc gccacagaaa caaccttgaa ggtgaagaat     600
   gattcagctt ctgctgacat cctggatgag ctgacagagc tgacagagct ggccaagagc     660
   gtcaccaaga atgatgttga tggcttcgag ttctacctca acaccttcca tgatgtgatg 720
   gtgggcaaca acctctttgg aagaagcacc ctcaagacag cttcagagct gatcgccaag 780
   gagaatgtca agacatctgg atcagaagtt ggaaatgtct acaacttcct ggtggtgctg 840
   acggcgctgc aagcaaaggc cttcctcacc ctcaccacct gccggaagct gctgggcctc 900
   gccgacatcg actacacctc catcatgaat gagcacctca acaaggagaa ggaggagttc 960
   agagtgaaca tcctccccat cctctccaac accttcagca accccaacta cgccaaggtg 1020
   aagggctcag atgaagatgc caagatgatt gtggaggcca agcctggcca tgctctggtg 1080
   ggcttcgaga tcagcaatga cagcatgacg gtgctgaagg tgtatgaagc aaagctgaag 1140
   cagaactacc aggtggacaa ggacagcctc tccgaggtga tctatggaga catggacaag 1200
   ctgctctgcc cagatcaatc agagaagatc tactacacca acaacatcgt ctttccaaat 1260
   gaatatgtca tcaccaagat cgacttcacc aagaagatga aaaccttgag atatgaagtc 1320
   accgccaaca gctatgattc ttcaactgga gagatcgacc tcaacaagaa gaaggtggag 1380
   agcagcaagg cagagtacag gacgctctcc gccaacaatg atggcgtcta catgccgctc 1440
   ggcgtcatct cagaaacctt cttgacgccc atcaatggct tcggcctcca agctgatgag 1500
   aacagcaggc tcatcaccct cacctgcaag agctacctca gggagctgct gctggcaaca 1560
   gatctctcca acaaggaaac aaagctgatt gttcctccaa acagcttcat cagcaacatc 1620
   gtggagaatg gaagcattga agaaggccac ctagagccat ggaaggccaa caacaagaat 1680
   gcatatgtgg accacaccgg cggcgtcaat ggcaccaagg cgctctatgt tcatgaagat 1740
   ggaggagttt cacagttcat gggagacaag ctgaagccaa aaacagaata tgtcatccag 1800
   tacaccgtca agggcaagcc aagcatccac ctcaaggatg agaacaccgg ctacatcctc 1860
   tacgaggaca ccaacaatga tttagaagat ttccaaacca tcaccaagag gttcaccact 1920
   ggaacagatc tgatgagggt gtacctcatc ctcaagagcc aaagtggtca tgaagcatgg 1980
   ggagacaact tcaccatcct ggagatcaag ccagcagaag ctctggtttc tccagagctc 2040
   atcaacccca acagctggat aacaacacaa ggagcttcaa tttctggtga caagctcttc 2100
   atctcccttg gaacaaatgg caccttccgc cagaacctct ccctcaacag ctacagcacc 2160
   tacagcatca gcttcaccgc ctcaggcccc ttcaatgtca ccgtcaggaa cagcagggag 2220
   gtgctgtatg aaaggaacaa cttgatgagc agcaccagcc acatctctgg agagttcaag 2280
   acagaaagca acaacaccgg cctctatgtg gagctctcaa gaagaagcgg cggcgccggc 2340
   cacatcagct tcgagaacat ctccatcaag 2370
<210> 3
   <211> 790
   <212> PRT
   <213> Bacillus thuringiensis
<400> 3
   Met Asn Met Asn Lys Asn Asn Thr Lys Leu Ser Ala Arg Ala Leu Pro
   1 5 10 15
   Ser Phe Ile Asp Tyr Phe Asn Gly Ile Tyr Gly Phe Ala Thr Gly Ile
   20 25 30
   Lys Asp Ile Met Asn Met Ile Phe Lys Thr Asp Thr Gly Gly Asp Leu
   35 40 45
   Thr Leu Asp Glu Ile Leu Lys Asn Gln Gln Leu Leu Asn Asp Ile Ser
   50 55 60
   Gly Lys Leu Asp Gly Val Asn Gly Ser Leu Asn Asp Leu Ile Ala Gln
   65 70 75 80
   Gly Asn Leu Asn Thr Glu Leu Ser Lys Glu Ile Leu Lys Ile Ala Asn
   85 90 95
   Glu Gln Asn Gln Val Leu Asn Asp Val Asn Asn Lys Leu Asp Ala Ile
   100 105 110
   Asn Thr Met Leu His Val Tyr Leu Pro Lys Ile Thr Ser Met Leu Ser
   115 120 125
   Asp Val Met Lys Pro Asn Tyr Ala Leu Ser Met Gln Ile Glu Tyr Leu
   130 135 140
   Ser Arg Gln Leu Gln Glu Ile Ser Asp Lys Leu Asp Ile Ile Asn Val
   145 150 155 160
   Asn Val Leu Ile Asn Ser Thr Leu Thr Glu Ile Thr Pro Ala Tyr Gln
   165 170 175
   Trp Ile Lys Tyr Val Asn Glu Lys Phe Glu Glu Leu Thr Phe Ala Thr
   180 185 190
   Glu Thr Thr Leu Lys Val Lys Asn Asp Ser Ala Ser Ala Asp Ile Leu
   195 200 205
   Asp Glu Leu Thr Glu Leu Thr Glu Leu Ala Lys Ser Val Thr Lys Asn
   210 215 220
   Asp Val Asp Gly Phe Glu Phe Tyr Leu Asn Thr Phe His Asp Val Met
   225 230 235 240
   Val Gly Asn Asn Leu Phe Gly Arg Ser Thr Leu Lys Thr Ala Ser Glu
   245 250 255
   Leu Ile Ala Lys Glu Asn Val Lys Thr Ser Gly Ser Glu Val Gly Asn
   260 265 270
   Val Tyr Asn Phe Leu Val Val Leu Thr Ala Leu Gln Ala Lys Ala Phe
           275                 280                 285
   Leu Thr Leu Thr Thr Cys Arg Lys Leu Leu Gly Leu Ala Asp Ile Asp
       290                 295                 300
   Tyr Thr Ser Ile Met Asn Glu His Leu Asn Lys Glu Lys Glu Glu Phe
   305                 310                 315                 320
   Arg Val Asn Ile Leu Pro Ile Leu Ser Asn Thr Phe Ser Asn Pro Asn
                   325                 330                 335
   Tyr Ala Lys Val Lys Gly Ser Asp Glu Asp Ala Lys Met Ile Val Glu
               340                 345                 350
   Ala Lys Pro Gly His Ala Leu Val Gly Phe Glu Ile Ser Asn Asp Ser
           355                 360                 365
   Met Thr Val Leu Lys Val Tyr Glu Ala Lys Leu Lys Gln Asn Tyr Gln
       370                 375                 380
   Val Asp Lys Asp Ser Leu Ser Glu Val Ile Tyr Gly Asp Met Asp Lys
   385                 390                 395                 400
   Leu Leu Cys Pro Asp Gln Ser Glu Lys Ile Tyr Tyr Thr Asn Asn Ile
                   405                 410                 415
   Val Phe Pro Asn Glu Tyr Val Ile Thr Lys Ile Asp Phe Thr Lys Lys
               420                 425                 430
   Met Lys Thr Leu Arg Tyr Glu Val Thr Ala Asn Ser Tyr Asp Ser Ser
           435                 440                 445
   Thr Gly Glu Ile Asp Leu Asn Lys Lys Lys Val Glu Ser Ser Lys Ala
       450                 455                 460
   Glu Tyr Arg Thr Leu Ser Ala Asn Asn Asp Gly Val Tyr Met Pro Leu
   465                 470                 475                 480
   Gly Val Ile Ser Glu Thr Phe Leu Thr Pro Ile Asn Gly Phe Gly Leu
                   485                 490                 495
   Gln Ala Asp Glu Asn Ser Arg Leu Ile Thr Leu Thr Cys Lys Ser Tyr
               500                 505                 510
   Leu Arg Glu Leu Leu Leu Ala Thr Asp Leu Ser Asn Lys Glu Thr Lys
           515                 520                 525
   Leu Ile Val Pro Pro Asn Ser Phe Ile Ser Asn Ile Val Glu Asn Gly
       530                 535                 540
   Ser Ile Glu Glu Gly His Leu Glu Pro Trp Lys Ala Asn Asn Lys Asn
   545 550 555 560
   Ala Tyr Val Asp His Thr Gly Gly Val Asn Gly Thr Lys Ala Leu Tyr
   565 570 575
   Val His Glu Asp Gly Gly Val Ser Gln Phe Met Gly Asp Lys Leu Lys
   580 585 590
   Pro Lys Thr Glu Tyr Val Ile Gln Tyr Thr Val Lys Gly Lys Pro Ser
   595 600 605
   Ile His Leu Lys Asp Glu Asn Thr Gly Tyr Ile Leu Tyr Glu Asp Thr
   610 615 620
   Asn Asn Asp Leu Glu Asp Phe Gln Thr Ile Thr Lys Arg Phe Thr Thr
   625 630 635 640
   Gly Thr Asp Leu Met Arg Val Tyr Leu Ile Leu Lys Ser Gln Ser Gly
   645 650 655
   His Glu Ala Trp Gly Asp Asn Phe Thr Ile Leu Glu Ile Lys Pro Ala
   660 665 670
   Glu Ala Leu Val Ser Pro Glu Leu Ile Asn Pro Asn Ser Trp Ile Thr
   675 680 685
   Thr Gln Gly Ala Ser Ile Ser Gly Asp Lys Leu Phe Ile Ser Leu Gly
   690 695 700
   Thr Asn Gly Thr Phe Arg Gln Asn Leu Ser Leu Asn Ser Tyr Ser Thr
   705 710 715 720
   Tyr Ser Ile Ser Phe Thr Ala Ser Gly Pro Phe Asn Val Thr Val Arg
   725 730 735
   Asn Ser Arg Glu Val Leu Tyr Glu Arg Asn Asn Leu Met Ser Ser Thr
   740 745 750
   Ser His Ile Ser Gly Glu Phe Lys Thr Glu Ser Asn Asn Thr Gly Leu
   755 760 765
   Tyr Val Glu Leu Ser Arg Arg Ser Gly Gly Ala Gly His Ile Ser Phe
   770 775 780
   Glu Asn Ile Ser Ile Lys
   785 790
<210> 4
   <211> 4
   <212> PRT
   <213> Artificial sequence
<220>
   <223> targeting peptide
<400> 4
   Lys Asp Glu Leu
   1

## Description

### REFERENCE TO SEQUENCE LISTING SUBMITTED ELECTRONICALLY

The official copy of the sequence listing is submitted electronically via EFS-Web as an ASCII formatted sequence listing with a file named "APA136001SEQLIST.txt", created on January 16, 2013, and having a size of 14 kilobytes and is filed concurrently with the specification. The sequence listing contained in this ASCII formatted document is part of the specification and is herein incorporated by reference in its entirety.

### FIELD OF THE INVENTION

This invention relates to the field of molecular biology. Genes that encode pesticidal proteins are disclosed. These proteins and the nucleic acid sequences that encode them are useful in preparing pesticidal formulations and in the production of transgenic pest- resistant plants.

### BACKGROUND OF THE FNVENTION

Corn rootworm is a significant agricultural pest. The Western corn rootworm, *Diabrotica virgifera virgifera,* is one of the most devastating corn rootworm species in North America, especially in the midwestern corn-growing areas such as Iowa. Both corn rootworm larvae and adults may damage corn plants. Newly hatched larvae feed primarily on root hairs and outer root tissue. As larvae grow and their food requirements increase, they burrow into the roots to feed. Larval damage is usually most severe after the secondary root system is well established and brace roots are developing. Root tips will appear brown and are often tunneled into and chewed back to the base of the plant. Larvae may be found tunneling into larger roots and occasionally in the plant crown. Crop rotation, early planting strategies, insecticides, and transgenic plants expressing toxins towards rootworm have been used to attempt to control their populations.

Transgenic crops engineered to produce insecticidal toxins derived from the bacterium *Bacillus thuringiensis* (Bt) were planted on more than 58 million hectares worldwide in 2010 (James C (2010) Global Status of Commercialized Biotech/GM Crops: 2010. ISAAA Brief No. 42. Ithaca, New York: ISAAA). Benefits of Bt crops include reduced use of harmful insecticides and regional suppression of some key agricultural pests (Carriere et al. (2003) Proc Natl Acad Sci U S A 100: 1519-1523; Cattaneo et al. (2006) Proc Natl Acad Sci U S A 103: 7571-7576; Huang et al. (2005) Science 308: 688-690; Wu (2008) Science 321: 1676-1678; and Hutchison et al. (2010) Science 330: 222-225). The western corn rootworm *Diabrotica virgifera virgifera* LeConte (Coleoptera: Chrysomelidae) is among the most serious pests of maize within the United States, with larval feeding on maize roots causing the majority of crop losses from this pest. Beginning in 2003, Bt maize was commercialized for control of western corn rootworm larvae and was rapidly adopted by farmers, constituting over 45% of maize crop in the United States during 2009 (James C (2009) Global Status of Commercialized Biotech/GM Crops: 2009. ISAAA Brief No. 41. Ithaca, New York: ISAAA; Environmental Protection Agency (2003) Biopesticides registration action document: event MON863 Bacillus thuringiensis Cry3Bbl Corn).

The refuge strategy is used in the United States and elsewhere to delay pest resistance to Bt crops (Gould F (1998) Annu Rev Entomol 43: 701-72). This strategy uses non-Bt host plants as a refuge for Bt susceptible genotypes. However, the western corn rootworm has repeatedly demonstrated its ability to adapt to pest management strategies (Gray et al. (2009) Annu Rev Entomol 54: 303-321). Examples include the evolution of resistance to conventional insecticides and the cultural practice of crop rotation (Meinke et al. (1998) J Econ Entomol 91: 594-600; Parimi et al. (2006) J Econ Entomol 25: 269-274; Levine et al. (2002) Am Entomol 48: 94-107). Further, Gassmann et al. (2011, PLoS ONE 6(7): e22629) recently reported that fields identified by farmers as having severe rootworm feeding injury to Bt maize contained populations of western corn rootworm that displayed significantly higher survival on Cry3Bbl maize in laboratory bioassays than did western corn rootworm from fields not associated with such feeding injury.

### SUMMARY OF INVENTION

Compositions and methods for conferring pesticidal activity to bacteria, plants, plant cells, tissues and seeds are described. Methods for killing or controlling a corn rootworm pest population, are provided. The methods comprise contacting the corn rootworm pest with a pesticidally-effective amount of a polypeptide comprising a corn rootworm toxin. In particular, the present invention provides a method for killing or controlling a corn rootworm pest population comprising contacting said population with a pesticidally-effective amount of a polypeptide comprising an amino acid sequence having at least 95% sequence identity to the amino acid sequence of SEQ ID NO:3, wherein the polypeptide has pesticidal activity against a corn rootworm pest. The coleopteran toxin comprises the amino acid sequence of SEQ ID NO:3, or pesticidally-effective variants or fragments thereof having at least 95% sequence identity to the amino acid sequence of SEQ ID NO: 3. In some embodiments, the method for protecting a plant or cell thereof from a corn rootworm pest population comprises expressing in a plant or cell thereof a nucleic acid encoding SEQ ID NO:3, or a variant or fragment thereof, wherein the nucleic acid is operably linked to a promoter capable of directing expression of the nucleic acid in a plant cell.

The present invention therefore also provides a method for protecting a corn plant from a corn rootworm pest, comprising expressing in a corn plant or cell thereof a nucleotide sequence operably linked to a promoter capable of directing expression of the nucleotide sequence in a plant cell, wherein said nucleotide sequence is selected from the group consisting of:
a) the nucleotide sequence set forth in SEQ ID NO: 1 or 2; and
b) a nucleotide sequence that encodes a polypeptide comprising an amino acid sequence having at least 95% sequence identity to the amino acid sequence of SEQ ID NO:3,
wherein said polypeptide has pesticidal activity against a corn rootworm pest.

The present invention also provides a method for increasing yield in a corn plant comprising growing in a field a corn plant of or a seed thereof having stably incorporated into its genome a DNA construct comprising a nucleotide sequence operably linked to a promoter capable of directing expression of the nucleotide sequence in a plant cell, wherein said nucleotide sequence is selected from the group consisting of:
a) the nucleotide sequence set forth in SEQ ID NO: 1 or 2; and
b) a nucleotide sequence that encodes a polypeptide comprising an amino acid sequence having at least 95% sequence identity to the amino acid sequence of SEQ ID NO:3, wherein said polypeptide has pesticidal activity against a corn rootworm pest;
wherein said field is infested with a corn rootworm pest.

The compositions and methods described are useful for the production of organisms with enhanced pest resistance or tolerance. These organisms and compositions comprising the organisms are desirable for agricultural purposes.

### BRIEF DESCRIPTION OF THE FIGURE

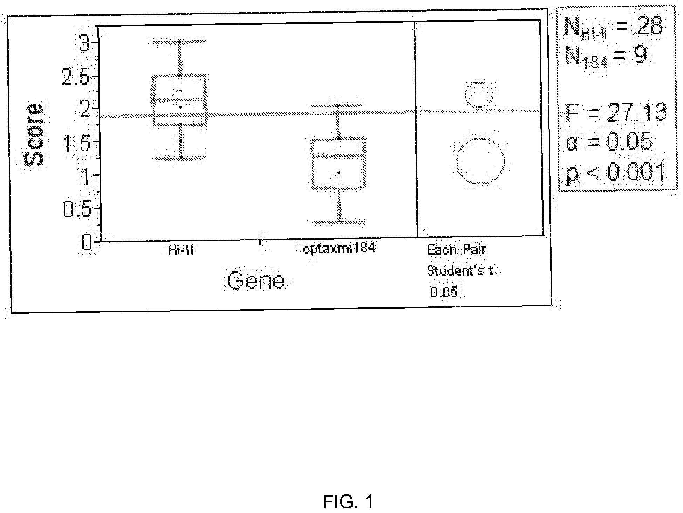

Figure 1 demonstrates the efficacy of Axmil84 in reducing root damage by Western corn rootworm.

### DETAILED DESCRIPTION

Methods for regulating pest resistance or tolerance in organisms, particularly plants or plant cells are described. By "resistance" is intended that the pest (e.g., insect) is killed upon ingestion or other contact with the polypeptides described. By "tolerance" is intended an impairment or reduction in the movement, feeding, reproduction, or other functions of the pest. The methods involve transforming organisms with a nucleotide sequence encoding a pesticidal protein of the invention. In particular, the nucleotide sequences of the invention are useful for preparing plants and microorganisms that possess pesticidal activity. The methods described herein are useful for controlling or killing corn rootworm pest populations and for producing compositions with pesticidal activity against corn rootworm pests.

By "pesticidal toxin" or "pesticidal protein" is intended a toxin that has toxic activity against one or more corn rootworm pests, including, but not limited to, Western corn rootworm (e.g., *Diabrotica virgifera virgifera*), Northern corn rootworm (e.g., *Diabrotica barberi*), and Southern corn rootworm (e.g., *Diabrotica undecimpunctata howardi*) pests, or a protein that has homology to such a protein. Pesticidal proteins include amino acid sequences deduced from the full-length nucleotide sequences disclosed herein, and amino acid sequences that are shorter than the full-length sequences, either due to the use of an alternate downstream start site, or due to processing that produces a shorter protein having pesticidal activity. Processing may occur in the organism the protein is expressed in, or in the pest after ingestion of the protein.

In specific embodiments, the pesticidal protein comprises an Axmil84 protein set forth in SEQ ID NO:3, as well as variants and fragments thereof that have at least 95% identity to the amino sequence of SEQ ID NO: 3 and pesticidal activity against a corn rootworm pest.

### Isolated Nucleic Acid Molecules, and Variants and Fragments Thereof

Isolated or recombinant nucleic acid molecules are disclosed comprising nucleotide sequences encoding pesticidal proteins and polypeptides or biologically active portions thereof, as well as nucleic acid molecules sufficient for use as hybridization probes to identify nucleic acid molecules encoding proteins with regions of sequence homology. Also disclosed herein are nucleotide sequences capable of hybridizing to the nucleotide sequences of the invention under stringent conditions as defined elsewhere herein. As used herein, the term "nucleic acid molecule" is intended to include DNA molecules (e.g., recombinant DNA, cDNA or genomic DNA) and RNA molecules (e.g., mRNA) and analogs of the DNA or RNA generated using nucleotide analogs. The nucleic acid molecule can be single-stranded or double-stranded, but preferably is double- stranded DNA.

The nucleotide sequence employed in the methods of the invention is one selected from the group consisting of:
a) the nucleotide sequence set forth in SEQ ID NO: 1 or 2; and
b) a nucleotide sequence that encodes a polypeptide comprising an amino acid sequence having at least 95% sequence identity to the amino acid sequence of SEQ ID NO:3,
wherein said polypeptide has pesticidal activity against a corn rootworm pest.

An "isolated" or "recombinant" nucleic acid sequence (or DNA) is used herein to refer to a nucleic acid sequence (or DNA) that is no longer in its natural environment, for example in an *in vitro* or in a recombinant bacterial or plant host cell. In some instances, an isolated or recombinant nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For purposes of the invention, "isolated" when used to refer to nucleic acid molecules excludes isolated chromosomes. For example, in various instances, the isolated delta- endotoxin encoding nucleic acid molecule can contain less than about 5kb, 4kb, 3kb, 2kb, 1kb, 0.5kb, or 0.1kb of nucleotide sequences that naturally flank the nucleic acid molecule in genomic DNA of the cell from which the nucleic acid is derived. In various instances, a delta-endotoxin protein that is substantially free of cellular material includes preparations of protein having less than about 30%, 20%, 10%, or 5% (by dry weight) of non-delta-endotoxin protein (also referred to herein as a "contaminating protein").

Nucleotide sequences encoding the proteins include the sequence set forth in SEQ ID NO: 1-2, and variants, fragments, and complements thereof. By "complement" is intended a nucleotide sequence that is sufficiently complementary to a given nucleotide sequence such that it can hybridize to the given nucleotide sequence to thereby form a stable duplex. The corresponding amino acid sequences for the pesticidal proteins encoded by these nucleotide sequences are set forth in SEQ ID NO:3 and polypeptides comprising an amino acid sequence having at least 95% sequence identity to the amino acid sequence of SEQ ID NO: 3 and which have pesticidal activity against a corn rootworm pest.

Nucleic acid molecules that are fragments of these nucleotide sequences encoding pesticidal proteins are also disclosed. By "fragment" is intended a portion of the nucleotide sequence encoding a pesticidal protein. A fragment of a nucleotide sequence may encode a biologically active portion of a pesticidal protein, or it may be a fragment that can be used as a hybridization probe or PCR primer using methods disclosed below. Nucleic acid molecules that are fragments of a nucleotide sequence encoding a pesticidal protein comprise at least about 50, 100, 200, 300, 400, 500, 600, 700, 800, 900, 1000, 1100, 1200, 1300, 1350, 1400 contiguous nucleotides, or up to the number of nucleotides present in a full-length nucleotide sequence encoding a pesticidal protein disclosed herein, depending upon the intended use. By "contiguous" nucleotides is intended nucleotide residues that are immediately adjacent to one another. Fragments of the nucleotide sequences will encode protein fragments that retain the biological activity of the pesticidal protein and, hence, retain pesticidal activity. Fragments of nucleotide sequences that may be employed in the invention are ones comprising a nucleotide sequence that encodes a polypeptide comprising an amino acid sequence having at least 95% sequence identity to the amino acid sequence of SEQ ID NO:3, wherein said polypeptide has pesticidal activity against a corn rootworm pest.

Thus, biologically-active fragments of the polypeptides disclosed herein are also disclosed any may be employed provided they comprise an amino acid sequence having at least 95% sequence identity to that of SEQ ID NO: 3 and have pesticidal activity against a corn rootworm pest.

By "retains activity" is intended that the fragment will have at least about 30%, at least about 50%, at least about 70%, 80%, 90%, 95% or higher of the pesticidal activity of the pesticidal protein. The pesticidal activity is activity against a corn rootworm pest. Methods for measuring pesticidal activity are well known in the art. See, for example, Czapla and Lang (1990) J. Econ. Entomol. 83:2480-2485; Andrews et al. (1988) Biochem. J. 252: 199-206; Marrone et al. (1985) J. of Economic Entomology 78:290-293; and U.S. Patent No. 5,743,477.

A fragment of a nucleotide sequence encoding a pesticidal protein that encodes a biologically active portion of a protein disclosed will encode at least about 15, 25, 30, 50, 75, 100, 125, 150, 175, 200, 250, 300, 350, 400, 450 contiguous amino acids, or up to the total number of amino acids present in a full-length pesticidal protein of the invention. In some embodiments, the fragment is a proteolytic cleavage fragment. For example, the proteolytic cleavage fragment may have an N-terminal or a C-terminal truncation of at least about 100 amino acids, about 120, about 130, about 140, about 150, or about 160 amino acids relative to SEQ ID NO:3. In some embodiments, the fragments encompassed herein result from the removal of the C-terminal crystallization domain, e.g., by proteolysis or by insertion of a stop codon in the coding sequence. A fragment employed in the methods of the invention is one comprising amino acid sequence having at least 95% sequence identity to the amino acid sequence of SEQ ID NO:3, wherein the polypeptide has pesticidal activity against a corn rootworm pest.

Preferred pesticidal proteins are encoded by a nucleotide sequence sufficiently identical to the nucleotide sequence of SEQ ID NO: 1-2, or the pesticidal proteins are sufficiently identical to the amino acid sequence set forth in SEQ ID NO:3. By "sufficiently identical" is intended an amino acid or nucleotide sequence that has at least 95%, 96%, 97%, 98%, 99% or greater sequence identity compared to a reference sequence using one of the alignment programs described herein using standard parameters. One of skill in the art will recognize that these values can be appropriately adjusted to determine corresponding identity of proteins encoded by two nucleotide sequences by taking into account codon degeneracy, amino acid similarity, reading frame positioning, and the like.

To determine the percent identity of two amino acid sequences or of two nucleic acids, the sequences are aligned for optimal comparison purposes. The percent identity between the two sequences is a function of the number of identical positions shared by the sequences (i.e., percent identity = number of identical positions/total number of positions (e.g., overlapping positions) x 100). In one embodiment, the two sequences are the same length. In another embodiment, the percent identity is calculated across the entirety of the reference sequence (i.e., the sequence disclosed herein as any of SEQ ID NO: 1-3). The percent identity between two sequences can be determined using techniques similar to those described below, with or without allowing gaps. In calculating percent identity, typically exact matches are counted. A gap, i.e. a position in an alignment where a residue is present in one sequence but not in the other, is regarded as a position with non-identical residues.

The determination of percent identity between two sequences can be accomplished using a mathematical algorithm. A nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the BLASTN and BLASTX programs of Altschul et al. (1990) J. Mol. Biol. 215:403. BLAST nucleotide searches can be performed with the BLASTN program, score = 100, wordlength = 12, to obtain nucleotide sequences homologous to pesticidal-like nucleic acid molecules of the invention. BLAST protein searches can be performed with the BLASTX program, score = 50, wordlength = 3, to obtain amino acid sequences homologous to pesticidal protein molecules of the invention. To obtain gapped alignments for comparison purposes, Gapped BLAST (in BLAST 2.0) can be utilized as described in Altschul et al. (1997) Nucleic Acids Res. 25:3389. Alternatively, PSI-Blast can be used to perform an iterated search that detects distant relationships between molecules. See Altschul *et al.* (1997) *supra.* When utilizing BLAST, Gapped BLAST, and PSI-Blast programs, the default parameters of the respective programs (e.g., BLASTX and BLASTN) can be used. Alignment may also be performed manually by inspection.

Another non-limiting example of a mathematical algorithm utilized for the comparison of sequences is the ClustalW algorithm (Higgins et al. (1994) Nucleic Acids Res. 22:4673-4680). ClustalW compares sequences and aligns the entirety of the amino acid or DNA sequence, and thus can provide data about the sequence conservation of the entire amino acid sequence. The ClustalW algorithm is used in several commercially available DNA/amino acid analysis software packages, such as the ALIGNX module of the Vector NTI Program Suite (Invitrogen Corporation, Carlsbad, CA). After alignment of amino acid sequences with ClustalW, the percent amino acid identity can be assessed. A non-limiting example of a software program useful for analysis of ClustalW alignments is GENEDOC™. GENEDOC™ (Karl Nicholas) allows assessment of amino acid (or DNA) similarity and identity between multiple proteins. Another non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG Wisconsin Genetics Software Package, Version 10 (available from Accelrys, Inc., 9685 Scranton Rd., San Diego, CA, USA). When utilizing the ALIGN program for comparing amino acid sequences, a PAM120 weight residue table, a gap length penalty of 12, and a gap penalty of 4 can be used.

Unless otherwise stated, GAP Version 10, which uses the algorithm of Needleman and Wunsch (1970) J. Mol. Biol. 48(3):443-453, will be used to determine sequence identity or similarity using the following parameters: % identity and % similarity for a nucleotide sequence using GAP Weight of 50 and Length Weight of 3, and the nwsgapdna.cmp scoring matrix; % identity or % similarity for an amino acid sequence using GAP weight of 8 and length weight of 2, and the BLOSUM62 scoring program. Equivalent programs may also be used. By "equivalent program" is intended any sequence comparison program that, for any two sequences in question, generates an alignment having identical nucleotide residue matches and an identical percent sequence identity when compared to the corresponding alignment generated by GAP Version 10.

The invention also encompasses variant nucleic acid molecules. "Variants" of the pesticidal protein encoding nucleotide sequences include those sequences that encode the pesticidal proteins disclosed herein but that differ conservatively because of the degeneracy of the genetic code as well as those that are sufficiently identical as discussed above. Naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the pesticidal proteins disclosed in the present invention as discussed below. Variant proteins encompassed by the present invention are biologically active, that is they continue to possess the desired biological activity of the native protein, that is, pesticidal activity. By "retains activity" is intended that the variant will have at least about 30%, at least about 50%, at least about 70%, or at least about 80% of the pesticidal activity of the native protein. Methods for measuring pesticidal activity are well known in the art. See, for example, Czapla and Lang (1990) J. Econ. Entomol. 83: 2480-2485; Andrews et al. (1988) Biochem. J. 252:199-206; Marrone et al. (1985) J. of Economic Entomology 78:290-293; and U.S. Patent No. 5,743,477.

The skilled artisan will further appreciate that changes can be introduced by mutation of the nucleotide sequences of the invention thereby leading to changes in the amino acid sequence of the encoded pesticidal proteins, without altering the biological activity of the proteins. Thus, variant isolated nucleic acid molecules can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention.

For example, conservative amino acid substitutions may be made at one or more, predicted, nonessential amino acid residues. A "nonessential" amino acid residue is a residue that can be altered from the wild-type sequence of a pesticidal protein without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Amino acid substitutions may be made in nonconserved regions that retain function. In general, such substitutions would not be made for conserved amino acid residues, or for amino acid residues residing within a conserved motif, where such residues are essential for protein activity. Examples of residues that are conserved and that may be essential for protein activity include, for example, residues that are identical between all proteins contained in an alignment of similar or related toxins to the sequences of the invention (e.g., residues that are identical in an alignment of homologous proteins). Examples of residues that are conserved but that may allow conservative amino acid substitutions and still retain activity include, for example, residues that have only conservative substitutions between all proteins contained in an alignment of similar or related toxins to the sequences of the invention (e.g., residues that have only conservative substitutions between all proteins contained in the alignment homologous proteins). However, one of skill in the art would understand that functional variants may have minor conserved or nonconserved alterations in the conserved residues.

Alternatively, variant nucleotide sequences can be made by introducing mutations randomly along all or part of the coding sequence, such as by saturation mutagenesis, and the resultant mutants can be screened for ability to confer pesticidal activity to identify mutants that retain activity. Following mutagenesis, the encoded protein can be expressed recombinantly, and the activity of the protein can be determined using standard assay techniques.

Using methods such as PCR, hybridization, and the like corresponding pesticidal sequences can be identified, such sequences having substantial identity to the sequences of the invention. See, for example, Sambrook and Russell (2001) Molecular Cloning: A Laboratory Manual. (Cold Spring Harbor Laboratory Press, Cold Spring Harbor, NY) and Innis, et al. (1990) PCR Protocols: A Guide to Methods and Applications (Academic Press, NY).

In a hybridization method, all or part of the pesticidal nucleotide sequence can be used to screen cDNA or genomic libraries. Methods for construction of such cDNA and genomic libraries are generally known in the art and are disclosed in Sambrook and Russell, 2001, *supra.* The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known pesticidal protein-encoding nucleotide sequence disclosed herein. Degenerate primers designed on the basis of conserved nucleotides or amino acid residues in the nucleotide sequence or encoded amino acid sequence can additionally be used. The probe typically comprises a region of nucleotide sequence that hybridizes under stringent conditions to at least about 12, at least about 25, at least about 50, 75, 100, 125, 150, 175, or 200 consecutive nucleotides of nucleotide sequence encoding a pesticidal protein disclosed herein or a fragment or variant thereof. Methods for the preparation of probes for hybridization are generally known in the art and are disclosed in Sambrook and Russell, 2001, *supra.*

For example, an entire pesticidal sequence disclosed herein, or one or more portions thereof, may be used as a probe capable of specifically hybridizing to corresponding pesticidal protein-like sequences and messenger RNAs. To achieve specific hybridization under a variety of conditions, such probes include sequences that are unique and are preferably at least about 10 nucleotides in length, or at least about 20 nucleotides in length. Such probes may be used to amplify corresponding pesticidal sequences from a chosen organism by PCR. This technique may be used to isolate additional coding sequences from a desired organism or as a diagnostic assay to determine the presence of coding sequences in an organism. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Cold Spring Harbor, New York).

Thus, also disclosed are probes for hybridization, as well as nucleotide sequences capable of hybridization to all or a portion of a nucleotide sequence disclosed herein (e.g., at least about 300 nucleotides, at least about 400, at least about 500, 1000, 1200, 1500, 2000, 2500, 3000, 3500, or up to the full length of a nucleotide sequence disclosed herein). Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tm, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Cold Spring Harbor, New York).

### Isolated Proteins and Variants and Fragments Thereof

Pesticidal proteins are also disclosed. By "pesticidal protein" is intended a protein having the amino acid sequence set forth in SEQ ID NO:3. Fragments, biologically active portions, and variants thereof are also provided, and may be used to practice the methods of the present invention provided that they comprise an amino acid sequence having at least 95% sequence identity to the amino acid sequence of SEQ ID NO:3, wherein the polypeptide has pesticidal activity against a corn rootworm pest. An "isolated protein" or a "recombinant protein" is used to refer to a protein that is no longer in its natural environment, for example in vitro or in a recombinant bacterial or plant host cell.

"Fragments" or "biologically active portions" include polypeptide fragments comprising amino acid sequences sufficiently identical to the amino acid sequence set forth in SEQ ID NO:3, and that exhibit pesticidal activity against a corn rootworm pest. A biologically active portion of a pesticidal protein can be a polypeptide that is, for example, 10, 25, 50, 100, 150, 200, 250, 300, 350, 400, 450, 500, 550, 600, 650, 700, 750, 800, 850, 900, 950, 1000, 1050, 1100, 1150, 1200, 1250, 1300, 1350, or more amino acids in length. Such biologically active portions can be prepared by recombinant techniques and evaluated for pesticidal activity. Methods for measuring pesticidal activity are well known in the art. See, for example, Czapla and Lang (1990) J. Econ. Entomol. 83:2480-2485; Andrews et al. (1988) Biochem. J. 252: 199-206; Marrone et al. (1985) J. of Economic Entomology 78:290-293; and U.S. Patent No. 5,743,477. As used here, a fragment comprises at least 8 contiguous amino acids of SEQ ID NO:3. Other fragments, however, are disclosed such as any fragment in the protein greater than about 10, 20, 30, 50, 100, 150, 200, 250, 300, 350, 400, 450, 500, 550, 600, 650, 700, 750 or more amino acids in length.

By "variants" is intended proteins or polypeptides having an amino acid sequence that is at least 95%, 96%, 97%, 98% or 99% identical to the amino acid sequence of any of SEQ ID NO:3. Variants also include polypeptides encoded by a nucleic acid molecule that hybridizes to the nucleic acid molecule of SEQ ID NO: 1-2, or a complement thereof, under stringent conditions. Variants include polypeptides that differ in amino acid sequence due to mutagenesis. Variant proteins employed by the present invention are biologically active, that is they continue to possess the desired biological activity of the native protein, that is, retaining pesticidal activity. In particular, they have at least 95% amino acid sequence identity to SEQ ID NO: 3 and pesticidal activity against a corn rootworm pest. In some embodiments, the variants have improved activity relative to the native protein. Methods for measuring pesticidal activity are well known in the art. See, for example, Czapla and Lang (1990) J. Econ. Entomol. 83:2480-2485; Andrews et al. (1988) Biochem. J. 252: 199-206; Marrone et al. (1985) J. of Economic Entomology 78:290-293; and U.S. Patent No. 5,743,477.

Bacterial genes, such as the *axmi* genes disclosed herein, quite often possess multiple methionine initiation codons in proximity to the start of the open reading frame. Often, translation initiation at one or more of these start codons will lead to generation of a functional protein. These start codons can include ATG codons. However, bacteria such as *Bacillus sp.* also recognize the codon GTG as a start codon, and proteins that initiate translation at GTG codons contain a methionine at the first amino acid. On rare occasions, translation in bacterial systems can initiate at a TTG codon, though in this event the TTG encodes a methionine. Furthermore, it is not often determined a *priori* which of these codons are used naturally in the bacterium. Thus, it is understood that use of one of the alternate methionine codons may also lead to generation of pesticidal proteins. These pesticidal proteins are disclosed and may be used in the methods of the present invention. It will be understood that, when expressed in plants, it will be necessary to alter the alternate start codon to ATG for proper translation.

In various instances, pesticidal proteins include amino acid sequences deduced from the full-length nucleotide sequences disclosed herein, and amino acid sequences that are shorter than the full-length sequences due to the use of an alternate downstream start site. Thus, the nucleotide sequence and/or vectors, host cells, and plants comprising the nucleotide sequence (and methods of making and using the nucleotide sequence) may comprise a nucleotide sequence encoding an amino acid sequence corresponding to residues 3-790 of SEQ ID NO:3, residues 36-790 of SEQ ID NO:3, or residues 38-790 of SEQ ID NO:3.

Antibodies to the polypeptides disclosed, or to variants or fragments thereof, are also disclosed. Methods for producing antibodies are well known in the art (see, for example, Harlow and Lane (1988) Antibodies: A Laboratory Manual, Cold Spring Harbor Laboratory, Cold Spring Harbor, NY; U.S. Patent No. 4,196,265).

Thus, also disclosed are antibodies, single-chain antigen binding molecules, or other proteins that specifically bind to one or more of the protein or peptide molecules disclosed and their homologs, fusions or fragments. In a particularly preferred instance, the antibody specifically binds to a protein having the amino acid sequence set forth in SEQ ID NO: 3 or a fragment thereof. In another instance, the antibody specifically binds to a fusion protein comprising an amino acid sequence selected from the amino acid sequence set forth in SEQ ID NO: 3 or a fragment thereof.

Antibodies may be used to quantitatively or qualitatively detect the protein or peptide molecules of the invention, or to detect post translational modifications of the proteins. As used herein, an antibody or peptide is said to "specifically bind" to a protein or peptide molecule if such binding is not competitively inhibited by the presence of non-related molecules.

### Altered or Improved Variants

It is recognized that DNA sequences of a pesticidal protein may be altered by various methods, and that these alterations may result in DNA sequences encoding proteins with amino acid sequences different than that encoded by a pesticidal protein disclosed herein. This protein may be altered in various ways including amino acid substitutions, deletions, truncations, and insertions of one or more amino acids of SEQ ID NO:3, including up to about 2, about 3, about 4, about 5, about 6, about 7, about 8, about 9, about 10, about 15, about 20, about 25, about 30, about 35, about 40, about 45, about 50, about 55, about 60, about 65, about 70, about 75, about 80, about 85, about 90, about 100, about 105, about 110,
about 115, about 120, about 125, about 130, about 135, about 140, about 145, about 150, about 155, or more amino acid substitutions, deletions or insertions. Methods for such manipulations are generally known in the art. For example, amino acid sequence variants of a pesticidal protein can be prepared by mutations in the DNA. This may also be accomplished by one of several forms of mutagenesis and/or in directed evolution. In some aspects, the changes encoded in the amino acid sequence will not substantially affect the function of the protein. Such variants will possess the desired pesticidal activity. However, it is understood that the ability of a pesticidal protein to confer pesticidal activity may be improved by the use of such techniques upon the compositions of this invention. For example, one may express a pesticidal protein in host cells that exhibit high rates of base misincorporation during DNA replication, such as XL-1 Red (Stratagene, La Jolla, CA). After propagation in such strains, one can isolate the DNA (for example by preparing plasmid DNA, or by amplifying by PCR and cloning the resulting PCR fragment into a vector), culture the pesticidal protein mutations in a non-mutagenic strain, and identify mutated genes with pesticidal activity, for example by performing an assay to test for pesticidal activity. Generally, the protein is mixed and used in feeding assays. See, for example Marrone et al. (1985) J. of Economic Entomology 78:290-293. Such assays can include contacting plants with one or more pests and determining the plant's ability to survive and/or cause the death of the pests. Examples of mutations that result in increased toxicity are found in Schnepf et al. (1998) Microbiol. Mol. Biol. Rev. 62:775-806.

Alternatively, alterations may be made to the protein sequence of many proteins at the amino or carboxy terminus without substantially affecting activity. This can include insertions, deletions, or alterations introduced by modem molecular methods, such as PCR, including PCR amplifications that alter or extend the protein coding sequence by virtue of inclusion of amino acid encoding sequences in the oligonucleotides utilized in the PCR amplification. Alternatively, the protein sequences added can include entire protein-coding sequences, such as those used commonly in the art to generate protein fusions. Such fusion proteins are often used to (1) increase expression of a protein of interest (2) introduce a binding domain, enzymatic activity, or epitope to facilitate either protein purification, protein detection, or other experimental uses known in the art (3) target secretion or translation of a protein to a subcellular organelle, such as the periplasmic space of Gram-negative bacteria, or the endoplasmic reticulum of eukaryotic cells, the latter of which often results in glycosylation of the protein.

Variant nucleotide and amino acid sequences of the present invention also encompass sequences derived from mutagenic and recombinogenic procedures such as DNA shuffling. With such a procedure, one or more different pesticidal protein coding regions can be used to create a new pesticidal protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between a pesticidal gene of the invention and other known pesticidal genes to obtain a new gene coding for a protein with an improved property of interest, such as an increased insecticidal activity. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

Domain swapping or shuffling is another mechanism for generating altered pesticidal proteins. Domains may be swapped between pesticidal proteins, resulting in hybrid or chimeric toxins with improved pesticidal activity or target spectrum. Methods for generating recombinant proteins and testing them for pesticidal activity are well known in the art (see, for example, Naimov et al. (2001) Appl. Environ. Microbiol. 67:5328-5330; de Maagd et al. (1996) Appl. Environ. Microbiol. 62:1537-1543; Ge et al. (1991) J. Biol. Chem. 266:17954-17958; Schnepf et al. (1990) J. Biol. Chem. 265:20923-20930; Rang et al. 91999) Appl. Environ. Microbiol. 65:2918-2925).

### Vectors

A pesticidal sequence of the invention may be provided in an expression cassette for expression in a plant of interest. By "plant expression cassette" is intended a DNA construct that is capable of resulting in the expression of a protein from an open reading frame in a plant cell. Typically these contain a promoter and a coding sequence. Often, such constructs will also contain a 3' untranslated region. Such constructs may contain a "signal sequence" or "leader sequence" to facilitate co-translational or post-translational transport of the peptide to certain intracellular structures such as the chloroplast (or other plastid), endoplasmic reticulum, or Golgi apparatus.

By "signal sequence" is intended a sequence that is known or suspected to result in cotranslational or post-translational peptide transport across the cell membrane. In eukaryotes, this typically involves secretion into the Golgi apparatus, with some resulting glycosylation. Insecticidal toxins of bacteria are often synthesized as protoxins, which are protolytically activated in the gut of the target pest (Chang (1987) Methods Enzymol. 153:507-516). In some embodiments of the present invention, the signal sequence is located in the native sequence, or may be derived from a sequence of the invention. By "leader sequence" is intended any sequence that when translated, results in an amino acid sequence sufficient to trigger co-translational transport of the peptide chain to a subcellular organelle. Thus, this includes leader sequences targeting transport and/or glycosylation by passage into the endoplasmic reticulum, passage to vacuoles, plastids including chloroplasts, mitochondria, and the like.

By "plant transformation vector" is intended a DNA molecule that is necessary for efficient transformation of a plant cell. Such a molecule may consist of one or more plant expression cassettes, and may be organized into more than one "vector" DNA molecule. For example, binary vectors are plant transformation vectors that utilize two non-contiguous DNA vectors to encode all requisite cis- and trans-acting functions for transformation of plant cells (Hellens and Mullineaux (2000) Trends in Plant Science 5:446-451). "Vector" refers to a nucleic acid construct designed for transfer between different host cells. "Expression vector" refers to a vector that has the ability to incorporate, integrate and express heterologous DNA sequences or fragments in a foreign cell. The cassette will include 5' and/or 3' regulatory sequences operably linked to a sequence of the invention. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

In various embodiments, the nucleotide sequence of the invention is operably linked to a promoter, e.g., a plant promoter. "Promoter" refers to a nucleic acid sequence that functions to direct transcription of a downstream coding sequence. The promoter together with other transcriptional and translational regulatory nucleic acid sequences (also termed "control sequences") are necessary for the expression of a DNA sequence of interest.

Such an expression cassette is provided with a plurality of restriction sites for insertion of the pesticidal sequence to be under the transcriptional regulation of the regulatory regions.

The expression cassette will include in the 5'-3' direction of transcription, a transcriptional and translational initiation region (i.e., a promoter), a DNA sequence of the invention, and a translational and transcriptional termination region (i.e., termination region) functional in plants. The promoter may be native or analogous, or foreign or heterologous, to the plant host and/or to the DNA sequence of the invention. Additionally, the promoter may be the natural sequence or alternatively a synthetic sequence. Where the promoter is "native" or "homologous" to the plant host, it is intended that the promoter is found in the native plant into which the promoter is introduced. Where the promoter is "foreign" or "heterologous" to the DNA sequence of the invention, it is intended that the promoter is not the native or naturally occurring promoter for the operably linked DNA sequence of the invention.

The termination region may be native with the transcriptional initiation region, may be native with the operably linked DNA sequence of interest, may be native with the plant host, or may be derived from another source (i.e., foreign or heterologous to the promoter, the DNA sequence of interest, the plant host, or any combination thereof). Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed host cell. That is, the genes can be synthesized using host cell-preferred codons for improved expression, or may be synthesized using codons at a host-preferred codon usage frequency. Generally, the GC content of the gene will be increased. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, U.S. Patent Publication No. 20090137409, and Murray et al. (1989) Nucleic Acids Res. 17:477-498.

In one embodiment, the pesticidal protein is targeted to the chloroplast for expression. In this manner, where the pesticidal protein is not directly inserted into the chloroplast, the expression cassette will additionally contain a nucleic acid encoding a transit peptide to direct the pesticidal protein to the chloroplasts. Such transit peptides are known in the art. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

The pesticidal gene to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831.

### Plant Transformation

Methods of the invention involve introducing a nucleotide construct into a plant. By "introducing" is intended to present to the plant the nucleotide construct in such a manner that the construct gains access to the interior of a cell of the plant. The methods of the invention do not require that a particular method for introducing a nucleotide construct to a plant is used, only that the nucleotide construct gains access to the interior of at least one cell of the plant. Methods for introducing nucleotide constructs into plants are known in the art including, but not limited to, stable transformation methods, transient transformation methods, and virus-mediated methods.

By "plant" is intended whole plants, plant organs (e.g., leaves, stems, roots, etc.), seeds, plant cells, propagules, embryos and progeny of the same. Plant cells can be differentiated or undifferentiated (e.g. callus, suspension culture cells, protoplasts, leaf cells, root cells, phloem cells, pollen).

"Transgenic plants" or "transformed plants" or "stably transformed" plants or cells or tissues refers to plants that have incorporated or integrated exogenous nucleic acid sequences or DNA fragments into the plant cell. These nucleic acid sequences include those that are exogenous, or not present in the untransformed plant cell, as well as those that may be endogenous, or present in the untransformed plant cell. "Heterologous" generally refers to the nucleic acid sequences that are not endogenous to the cell or part of the native genome in which they are present, and have been added to the cell by infection, transfection, microinjection, electroporation, microprojection, or the like.

The transgenic plants of the invention express one or more of the novel toxin sequences disclosed herein. In various embodiments, the transgenic plant further comprises one or more additional genes for insect resistance (e.g., Cry1, such as members of the Cry1A, Cry1B, Cry1C, Cry1D, Cry1E, and Cry1F families; Cry2, such as members of the Cry2A family; Cry9, such as members of the Cry9A, Cry9B, Cry9C, Cry9D, Cry9E, and Cry9F families; etc.). It will be understood by one of skill in the art that the transgenic plant may comprise any gene imparting an agronomic trait of interest.

Transformation of plant cells can be accomplished by one of several techniques known in the art. The pesticidal gene of the invention may be modified to obtain or enhance expression in plant cells. Typically a construct that expresses such a protein would contain a promoter to drive transcription of the gene, as well as a 3' untranslated region to allow transcription termination and polyadenylation. The organization of such constructs is well known in the art. In some instances, it may be useful to engineer the gene such that the resulting peptide is secreted, or otherwise targeted within the plant cell. For example, the gene can be engineered to contain a signal peptide to facilitate transfer of the peptide to the endoplasmic reticulum. It may also be preferable to engineer the plant expression cassette to contain an intron, such that mRNA processing of the intron is required for expression.

Typically this "plant expression cassette" will be inserted into a "plant transformation vector". This plant transformation vector may be comprised of one or more DNA vectors needed for achieving plant transformation. For example, it is a common practice in the art to utilize plant transformation vectors that are comprised of more than one contiguous DNA segment. These vectors are often referred to in the art as "binary vectors." Binary vectors as well as vectors with helper plasmids are most often used for *Agrobacterium*-mediated transformation, where the size and complexity of DNA segments needed to achieve efficient transformation is quite large, and it is advantageous to separate functions onto separate DNA molecules. Binary vectors typically contain a plasmid vector that contains the cis-acting sequences required for T-DNA transfer (such as left border and right border), a selectable marker that is engineered to be capable of expression in a plant cell, and a "gene of interest" (a gene engineered to be capable of expression in a plant cell for which generation of transgenic plants is desired). Also present on this plasmid vector are sequences required for bacterial replication. The cis-acting sequences are arranged in a fashion to allow efficient transfer into plant cells and expression therein. For example, the selectable marker gene and the pesticidal gene are located between the left and right borders. Often a second plasmid vector contains the trans-acting factors that mediate T-DNA transfer from *Agrobacterium* to plant cells. This plasmid often contains the virulence functions (Vir genes) that allow infection of plant cells by *Agrobacterium,* and transfer of DNA by cleavage at border sequences and vir-mediated DNA transfer, as is understood in the art (Hellens and Mullineaux (2000) Trends in Plant Science 5:446-451). Several types of *Agrobacterium* strains (e.g. LBA4404, GV3101, EHA101, EHA105, etc.) can be used for plant transformation. The second plasmid vector is not necessary for transforming the plants by other methods such as microprojection, microinjection, electroporation, polyethylene glycol, etc.

In general, plant transformation methods involve transferring heterologous DNA into target plant cells (e.g. immature or mature embryos, suspension cultures, undifferentiated callus, protoplasts, etc.), followed by applying a maximum threshold level of appropriate selection (depending on the selectable marker gene) to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent. The shoots are then transferred to a selective rooting medium for recovering rooted shoot or plantlet. The transgenic plantlet then grows into a mature plant and produces fertile seeds (e.g. Hiei et al. (1994) The Plant Journal 6:271-282; Ishida et al. (1996) Nature Biotechnology 14:745-750). Explants are typically transferred to a fresh supply of the same medium and cultured routinely. A general description of the techniques and methods for generating transgenic plants are found in Ayres and Park (1994) Critical Reviews in Plant Science 13:219-239 and Bommineni and Jauhar (1997) Maydica 42:107-120. Since the transformed material contains many cells; both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants.

Transformation protocols as well as protocols for introducing nucleotide sequences into plants may vary depending on the type of plant or plant cell, i.e., monocot or dicot, targeted for transformation. Generation of transgenic plants may be performed by one of several methods, including, but not limited to, microinjection, electroporation, direct gene transfer, introduction of heterologous DNA by *Agrobacterium* into plant cells (*Agrobacterium*-mediated transformation), bombardment of plant cells with heterologous foreign DNA adhered to particles, ballistic particle acceleration, aerosol beam transformation (U.S. Published Application No. 20010026941; U.S. Patent No. 4,945,050; International Publication No. WO 91/00915; U.S. Published Application No. 2002015066), Lec1 transformation, and various other non-particle direct-mediated methods to transfer DNA.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

Following integration of heterologous foreign DNA into plant cells, one then applies a maximum threshold level of appropriate selection in the medium to kill the untransformed cells and separate and proliferate the putatively transformed cells that survive from this selection treatment by transferring regularly to a fresh medium. By continuous passage and challenge with appropriate selection, one identifies and proliferates the cells that are transformed with the plasmid vector. Molecular and biochemical methods can then be used to confirm the presence of the integrated heterologous gene of interest into the genome of the transgenic plant.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a nucleotide construct of the invention, for example, an expression cassette of the invention, stably incorporated into their genome.

### Evaluation of Plant Transformation

Following introduction of heterologous foreign DNA into plant cells, the transformation or integration of heterologous gene in the plant genome is confirmed by various methods such as analysis of nucleic acids, proteins and metabolites associated with the integrated gene.

PCR analysis is a rapid method to screen transformed cells, tissue or shoots for the presence of incorporated gene at the earlier stage before transplanting into the soil (Sambrook and Russell (2001) Molecular Cloning: A Laboratory Manual. Cold Spring Harbor Laboratory Press, Cold Spring Harbor, NY). PCR is carried out using oligonucleotide primers specific to the gene of interest or *Agrobacterium* vector background, etc.

Plant transformation may be confirmed by Southern blot analysis of genomic DNA (Sambrook and Russell, 2001, *supra*). In general, total DNA is extracted from the transformant, digested with appropriate restriction enzymes, fractionated in an agarose gel and transferred to a nitrocellulose or nylon membrane. The membrane or "blot" is then probed with, for example, radiolabeled ³²P target DNA fragment to confirm the integration of introduced gene into the plant genome according to standard techniques (Sambrook and Russell, 2001, *supra*).

In Northern blot analysis, RNA is isolated from specific tissues of transformant, fractionated in a formaldehyde agarose gel, and blotted onto a nylon filter according to standard procedures that are routinely used in the art (Sambrook and Russell, 2001, *supra*). Expression of RNA encoded by the pesticidal gene is then tested by hybridizing the filter to a radioactive probe derived from a pesticidal gene, by methods known in the art (Sambrook and Russell, 2001, *supra*).

Western blot, biochemical assays and the like may be carried out on the transgenic plants to confirm the presence of protein encoded by the pesticidal gene by standard procedures (Sambrook and Russell, 2001, *supra*) using antibodies that bind to one or more epitopes present on the pesticidal protein.

### Pesticidal Activity in Plants

In another aspect of the invention, one may generate transgenic plants expressing a pesticidal protein that has pesticidal activity. Methods described above by way of example may be utilized to generate transgenic plants, but the manner in which the transgenic plant cells are generated is not critical to this invention. Methods known or described in the art such as *Agrobacterium*-mediated transformation, biolistic transformation, and non-particle-mediated methods may be used at the discretion of the experimenter. Plants expressing a pesticidal protein may be isolated by common methods described in the art, for example by transformation of callus, selection of transformed callus, and regeneration of fertile plants from such transgenic callus. In such process, one may use any gene as a selectable marker so long as its expression in plant cells confers ability to identify or select for transformed cells.

A number of markers have been developed for use with plant cells, such as resistance to chloramphenicol, the aminoglycoside G418, hygromycin, or the like. Other genes that encode a product involved in chloroplast metabolism may also be used as selectable markers. For example, genes that provide resistance to plant herbicides such as glyphosate, bromoxynil, or imidazolinone may find particular use. Such genes have been reported (Stalker et al. (1985) J. Biol. Chem. 263:6310-6314 (bromoxynil resistance nitrilase gene); and Sathasivan et al. (1990) Nucl. Acids Res. 18:2188 (AHAS imidazolinone resistance gene). Additionally, the genes disclosed herein are useful as markers to assess transformation of bacterial or plant cells. Methods for detecting the presence of a transgene in a plant, plant organ (e.g., leaves, stems, roots, etc.), seed, plant cell, propagule, embryo or progeny of the same are well known in the art. In one embodiment, the presence of the transgene is detected by testing for pesticidal activity.

Fertile plants expressing a pesticidal protein may be tested for pesticidal activity, and the plants showing optimal activity selected for further breeding. Methods are available in the art to assay for pest activity. Generally, the protein is mixed and used in feeding assays. See, for example Marrone et al. (1985) J. of Economic Entomology 78:290-293.

The present invention may be used for transformation of any plant species, including, but not limited to, monocots and dicots. Examples of plants of interest include, but are not limited to, corn (maize), sorghum, wheat, sunflower, tomato, crucifers, peppers, potato, cotton, rice, soybean, sugarbeet, sugarcane, tobacco, barley, and oilseed rape, *Brassica* sp., alfalfa, rye, millet, safflower, peanuts, sweet potato, cassava, coffee, coconut, pineapple, citrus trees, cocoa, tea, banana, avocado, fig, guava, mango, olive, papaya, cashew, macadamia, almond, oats, vegetables, ornamentals, and conifers.

Vegetables include, but are not limited to, tomatoes, lettuce, green beans, lima beans, peas, and members of the genus *Curcumis* such as cucumber, cantaloupe, and musk melon. Ornamentals include, but are not limited to, azalea, hydrangea, hibiscus, roses, tulips, daffodils, petunias, carnation, poinsettia, and chrysanthemum. Preferably, plants of the present invention are crop plants (for example, maize, sorghum, wheat, sunflower, tomato, crucifers, peppers, potato, cotton, rice, soybean, sugarbeet, sugarcane, tobacco, barley, oilseed rape., etc.).

### Use in Pesticidal Control

General methods for employing strains comprising a nucleotide sequence disclosed herein, or a variant thereof, in pest control or in engineering other organisms as pesticidal agents are known in the art. See, for example U.S. Patent No. 5,039,523 and EP 0480762A2.

The *Bacillus* strains containing a nucleotide sequence disclosed herein, or a variant thereof, or the microorganisms that have been genetically altered to contain a pesticidal gene disclosed herein and protein may be used for protecting agricultural crops and products from pests. In one aspect of the invention, whole, i.e., unlysed, cells of a toxin (pesticide)-producing organism are treated with reagents that prolong the activity of the toxin produced in the cell when the cell is applied to the environment of target pest(s).

Alternatively, the pesticide is produced by introducing a pesticidal gene into a cellular host. Expression of the pesticidal gene results, directly or indirectly, in the intracellular production and maintenance of the pesticide. In one aspect of this invention, these cells are then treated under conditions that prolong the activity of the toxin produced in the cell when the cell is applied to the environment of the target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein. Alternatively, one may formulate the cells expressing a gene of this invention such as to allow application of the resulting material as a pesticide.

The active ingredients of the present invention are normally applied in the form of compositions and can be applied to the crop area or plant to be treated, simultaneously or in succession, with other compounds. These compounds can be fertilizers, weed killers, cryoprotectants, surfactants, detergents, pesticidal soaps, dormant oils, polymers, and/or time-release or biodegradable carrier formulations that permit long-term dosing of a target area following a single application of the formulation. They can also be selective herbicides, chemical insecticides, virucides, microbicides, amoebicides, pesticides, fungicides, bacteriocides, nematocides, molluscicides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. Likewise the formulations may be prepared into edible "baits" or fashioned into pest "traps" to permit feeding or ingestion by a target pest of the pesticidal formulation.

Methods of applying an active ingredient of the present invention or an agrochemical composition of the present invention that contains at least one of the pesticidal proteins produced by the bacterial strains of the present invention include leaf application, seed coating and soil application. The number of applications and the rate of application depend on the intensity of infestation by the corresponding pest.

The composition may be formulated as a powder, dust, pellet, granule, spray, emulsion, colloid, solution, or such like, and may be prepared by such conventional means as desiccation, lyophilization, homogenation, extraction, filtration, centrifugation, sedimentation, or concentration of a culture of cells comprising the polypeptide. In all such compositions that contain at least one such pesticidal polypeptide, the polypeptide may be present in a concentration of from about 1% to about 99% by weight.

Coleopteran pests may be killed or reduced in numbers in a given area by the methods of the invention, or may be prophylactically applied to an environmental area to prevent infestation by a susceptible pest. Preferably the pest ingests, or is contacted with, a pesticidally-effective amount of the polypeptide. By "pesticidally-effective amount" is intended an amount of the pesticide that is able to bring about death to at least one pest, or to noticeably reduce pest growth, feeding, or normal physiological development. This amount will vary depending on such factors as, for example, the specific target pests to be controlled, the specific environment, location, plant, crop, or agricultural site to be treated, the environmental conditions, and the method, rate, concentration, stability, and quantity of application of the pesticidally-effective polypeptide composition. The formulations may also vary with respect to climatic conditions, environmental considerations, and/or frequency of application and/or severity of pest infestation.

The pesticide compositions described may be made by formulating either the bacterial cell, the crystal and/or the spore suspension, or the isolated protein component with the desired agriculturally-acceptable carrier. The compositions may be formulated prior to administration in an appropriate means such as lyophilized, freeze-dried, desiccated, or in an aqueous carrier, medium or suitable diluent, such as saline or other buffer. The formulated compositions may be in the form of a dust or granular material, or a suspension in oil (vegetable or mineral), or water or oil/water emulsions, or as a wettable powder, or in combination with any other carrier material suitable for agricultural application. Suitable agricultural carriers can be solid or liquid and are well known in the art. The term "agriculturally-acceptable carrier" covers all adjuvants, inert components, dispersants, surfactants, tackifiers, binders, etc. that are ordinarily used in pesticide formulation technology; these are well known to those skilled in pesticide formulation. The formulations may be mixed with one or more solid or liquid adjuvants and prepared by various means, e.g., by homogeneously mixing, blending and/or grinding the pesticidal composition with suitable adjuvants using conventional formulation techniques. Suitable formulations and application methods are described in U.S. Patent No. 6,468,523.

Examples of corn rootworm pests include, e.g., Western corn rootworm (e.g., *Diabrotica virgifera virgifera*), Northern corn rootworm (e.g., *Diabrotica barberi*), and Southern corn rootworm (e.g., *Diabrotica undecimpunctata howardi*).

### Methods for Increasing Plant Yield

Methods for increasing plant yield are provided. The methods comprise providing a plant or plant cell expressing a polynucleotide encoding the pesticidal polypeptide sequence disclosed herein and growing the plant or a seed thereof in a field infested with (or susceptible to infestation by) a pest against which said polypeptide has pesticidal activity. In particular, the present invention provides a method for increasing yield in a corn plant comprising growing in a field a corn plant of or a seed thereof having stably incorporated into its genome a DNA construct comprising a nucleotide sequence operably linked to a promoter capable of directing expression of the nucleotide sequence in a plant cell, wherein said nucleotide sequence is selected from the group consisting of:
a) the nucleotide sequence set forth in SEQ ID NO: 1 or 2; and
b) a nucleotide sequence that encodes a polypeptide comprising an amino acid sequence having at least 95% sequence identity to the amino acid sequence of SEQ ID NO:3, wherein said polypeptide has pesticidal activity against a corn rootworm pest;
wherein said field is infested with a corn rootworm pest. In some embodiments, the Axmil84 polypeptide described herein has pesticidal activity against a corn rootworm pest, and said field is infested with said corn rootworm pest. In various embodiments, the corn rootworm pest is Western corn rootworm. As defined herein, the "yield" of the plant refers to the quality and/or quantity of biomass produced by the plant. By "biomass" is intended any measured plant product. An increase in biomass production is any improvement in the yield of the measured plant product. Increasing plant yield has several commercial applications. For example, increasing plant leaf biomass may increase the yield of leafy vegetables for human or animal consumption. Additionally, increasing leaf biomass can be used to increase production of plant-derived pharmaceutical or industrial products. An increase in yield can comprise any statistically significant increase including, but not limited to, at least a 1% increase, at least a 3% increase, at least a 5% increase, at least a 10% increase, at least a 20% increase, at least a 30%, at least a 50%, at least a 70%, at least a 100% or a greater increase in yield compared to a plant not expressing the pesticidal sequence. In specific methods, plant yield is increased as a result of improved pest resistance of a plant expressing a pesticidal protein disclosed herein. Expression of the pesticidal protein results in a reduced ability of a pest to infest or feed.

The plants can also be treated with one or more chemical compositions, including one or more herbicide, insecticides, or fungicides. Exemplary chemical compositions include: Fruits/Vegetables Herbicides: Atrazine, Bromacil, Diuron, Glyphosate, Linuron, Metribuzin, Simazine, Trifluralin, Fluazifop, Glufosinate, Halosulfuron Gowan, Paraquat, Propyzamide, Sethoxydim, Butafenacil, Halosulfuron, Indaziflam; Fruits/Vegetables Insecticides: Aldicarb , Bacillus thuriengiensis, Carbaryl, Carbofuran, Chlorpyrifos, Cypermethrin, Deltamethrin, Abamectin, Cyfluthrin/beta-cyfluthrin, Esfenvalerate, Lambda-cyhalothrin, Acequinocyl, Bifenazate, Methoxyfenozide, Novaluron, Chromafenozide, Thiacloprid, Dinotefuran, Fluacrypyrim, Spirodiclofen, Gamma-cyhalothrin, Spiromesifen, Spinosad, Rynaxypyr, Cyazypyr, Triflumuron,Spirotetramat, Imidacloprid, Flubendiamide, Thiodicarb, Metaflumizone, Sulfoxaflor, Cyflumetofen, Cyanopyrafen, Clothianidin, Thiamethoxam, Spinotoram, Thiodicarb, Flonicamid, Methiocarb, Emamectin-benzoate, Indoxacarb, Fenamiphos, Pyriproxifen, Fenbutatin-oxid; Fruits/Vegetables Fungicides: Ametoctradin, Azoxystrobin, Benthiavalicarb, Boscalid, Captan, Carbendazim, Chlorothalonil, Copper, Cyazofamid, Cyflufenamid, Cymoxanil, Cyproconazole, Cyprodinil, Difenoconazole, Dimetomorph, Dithianon, Fenamidone, Fenhexamid, Fluazinam, Fludioxonil, Fluopicolide, Fluopyram, Fluoxastrobin, Fluxapyroxad, Folpet, Fosetyl, Iprodione, Iprovalicarb, Isopyrazam, Kresoxim-methyl, Mancozeb, Mandipropamid, Metalaxyl/mefenoxam, Metiram, Metrafenone, Myclobutanil, Penconazole, Penthiopyrad, Picoxystrobin, Propamocarb, Propiconazole, Propineb, Proquinazid, Prothioconazole, Pyraclostrobin, Pyrimethanil, Quinoxyfen, Spiroxamine, Sulphur, Tebuconazole, Thiophanate-methyl, Trifloxystrobin; Cereals Herbicides:
2.4-D, Amidosulfuron, Bromoxynil, Carfentrazone-E, Chlorotoluron, Chlorsulfuron, Clodinafop-P, Clopyralid, Dicamba, Diclofop-M, Diflufenican, Fenoxaprop, Florasulam, Flucarbazone-NA, Flufenacet, Flupyrosulfuron-M, Fluroxypyr, Flurtamone, Glyphosate, Iodosulfuron, Ioxynil, Isoproturon, MCPA, Mesosulfuron, Metsulfuron, Pendimethalin, Pinoxaden, Propoxycarbazone, Prosulfocarb, Pyroxsulam, Sulfosulfuron, Thifensulfuron, Tralkoxydim, Triasulfuron, Tribenuron, Trifluralin, Tritosulfuron; Cereals Fungicides: Azoxystrobin, Bixafen, Boscalid, Carbendazim, Chlorothalonil, Cyflufenamid, Cyproconazole, Cyprodinil, Dimoxystrobin, Epoxiconazole, Fenpropidin, Fenpropimorph, Fluopyram, Fluoxastrobin, Fluquinconazole, Fluxapyroxad, Isopyrazam, Kresoxim-methyl, Metconazole, Metrafenone, Penthiopyrad, Picoxystrobin, Prochloraz, Propiconazole, Proquinazid, Prothioconazole, Pyraclostrobin, Quinoxyfen, Spiroxamine, Tebuconazole, Thiophanate-methyl, Trifloxystrobin; Cereals Insecticides: Dimethoate, Lambda-cyhalthrin, Deltamethrin, alpha-Cypermethrin, β-cyfluthrin, Bifenthrin, Imidacloprid, Clothianidin, Thiamethoxam, Thiacloprid, Acetamiprid, Dinetofuran, Clorphyriphos, Pirimicarb, Methiocarb, Sulfoxaflor; Maize Herbicides: Atrazine, Alachlor, Bromoxynil, Acetochlor, Dicamba, Clopyralid, (S-)Dimethenamid, Glufosinate, Glyphosate, Isoxaflutole, (S-)Metolachlor, Mesotrione, Nicosulfuron, Primisulfuron, Rimsulfuron, Sulcotrione, Foramsulfuron, Topramezone, Tembotrione, Saflufenacil, Thiencarbazone, Flufenacet, Pyroxasulfon; Maize Insecticides: Carbofuran, Chlorpyrifos, Bifenthrin, Fipronil, Imidacloprid, Lambda-Cyhalothrin, Tefluthrin, Terbufos, Thiamethoxam, Clothianidin, Spiromesifen, Flubendiamide, Triflumuron, Rynaxypyr, Deltamethrin, Thiodicarb, β-Cyfluthrin, Cypermethrin, Bifenthrin, Lufenuron, Tebupirimphos, Ethiprole, Cyazypyr, Thiacloprid, Acetamiprid, Dinetofuran, Avermectin; Maize Fungicides: Azoxystrobin, Bixafen, Boscalid, Cyproconazole, Dimoxystrobin, Epoxiconazole, Fenitropan, Fluopyram, Fluoxastrobin, Fluxapyroxad, Isopyrazam, Metconazole, Penthiopyrad, Picoxystrobin, Propiconazole, Prothioconazole, Pyraclostrobin, Tebuconazole, Trifloxystrobin; Rice Herbicides: Butachlor, Propanil, Azimsulfuron, Bensulfuron, Cyhalofop, Daimuron, Fentrazamide, Imazosulfuron, Mefenacet, Oxaziclomefone, Pyrazosulfuron, Pyributicarb, Quinclorac, Thiobencarb, Indanofan, Flufenacet, Fentrazamide, Halosulfuron, Oxaziclomefone, Benzobicyclon, Pyriftalid, Penoxsulam, Bispyribac, Oxadiargyl, Ethoxysulfuron, Pretilachlor, Mesotrione, Tefuryltrione, Oxadiazone, Fenoxaprop, Pyrimisulfan; Rice Insecticides: Diazinon, Fenobucarb, Benfuracarb, Buprofezin, Dinotefuran, Fipronil, Imidacloprid, Isoprocarb, Thiacloprid, Chromafenozide, Clothianidin, Ethiprole, Flubendiamide, Rynaxypyr, Deltamethrin, Acetamiprid, Thiamethoxam, Cyazypyr, Spinosad, Spinotoram, Emamectin-Benzoate, Cypermethrin, Chlorpyriphos, Etofenprox, Carbofuran, Benfuracarb, Sulfoxaflor; Rice Fungicides: Azoxystrobin, Carbendazim, Carpropamid, Diclocymet, Difenoconazole, Edifenphos, Ferimzone, Gentamycin, Hexaconazole, Hymexazol, Iprobenfos (IBP), Isoprothiolane, Isotianil, Kasugamycin, Mancozeb, Metominostrobin, Orysastrobin, Pencycuron, Probenazole, Propiconazole, Propineb, Pyroquilon, Tebuconazole, Thiophanate-methyl, Tiadinil, Tricyclazole, Trifloxystrobin, Validamycin; Cotton Herbicides: Diuron, Fluometuron, MSMA, Oxyfluorfen, Prometryn, Trifluralin, Carfentrazone, Clethodim, Fluazifop-butyl, Glyphosate, Norflurazon, Pendimethalin, Pyrithiobac-sodium, Trifloxysulfuron, Tepraloxydim, Glufosinate, Flumioxazin, Thidiazuron; Cotton Insecticides: Acephate, Aldicarb, Chlorpyrifos, Cypermethrin, Deltamethrin, Abamectin, Acetamiprid, Emamectin Benzoate, Imidacloprid, Indoxacarb, Lambda-Cyhalothrin, Spinosad, Thiodicarb, Gamma-Cyhalothrin, Spiromesifen, Pyridalyl, Flonicamid
Flubendiamide, Triflumuron,Rynaxypyr,Beta-Cyfluthrin,Spirotetramat, Clothianidin, Thiamethoxam, Thiacloprid, Dinetofuran, Flubendiamide, Cyazypyr, Spinosad, Spinotoram, gamma Cyhalothrin, 4-[[(6-Chlorpyridin-3-yl)methyl](2,2-difluorethyl)amino]furan-2(5H)-on, Thiodicarb, Avermectin, Flonicamid, Pyridalyl, Spiromesifen, Sulfoxaflor; Cotton Fungicides: Azoxystrobin, Bixafen, Boscalid, Carbendazim, Chlorothalonil, Copper, Cyproconazole, Difenoconazole, Dimoxystrobin, Epoxiconazole, Fenamidone, Fluazinam, Fluopyram, Fluoxastrobin, Fluxapyroxad, Iprodione, Isopyrazam, Isotianil, Mancozeb, Maneb, Metominostrobin, Penthiopyrad, Picoxystrobin, Propineb, Prothioconazole, Pyraclostrobin, Quintozene, Tebuconazole, Tetraconazole, Thiophanate-methyl, Trifloxystrobin; Soybean Herbicides: Alachlor, Bentazone, Trifluralin, Chlorimuron-Ethyl, Cloransulam-Methyl, Fenoxaprop, Fomesafen, Fluazifop, Glyphosate, Imazamox, Imazaquin, Imazethapyr, (S-)Metolachlor, Metribuzin, Pendimethalin, Tepraloxydim, Glufosinate; Soybean Insecticides: Lambda-cyhalothrin, Methomyl, Imidacloprid, Clothianidin, Thiamethoxam, Thiacloprid, Acetamiprid, Dinetofuran, Flubendiamide, Rynaxypyr, Cyazypyr, Spinosad, Spinotoram, Emamectin-Benzoate, Fipronil, Ethiprole, Deltamethrin, β-Cyfluthrin, gamma and lambda Cyhalothrin, 4-[[(6-Chlorpyridin-3-yl)methyl](2,2-difluorethyl)amino]furan-2(5H)-on, Spirotetramat, Spinodiclofen, Triflumuron, Flonicamid, Thiodicarb, beta-Cyfluthrin; Soybean Fungicides: Azoxystrobin, Bixafen, Boscalid, Carbendazim, Chlorothalonil, Copper, Cyproconazole, Difenoconazole, Dimoxystrobin, Epoxiconazole, Fluazinam, Fluopyram, Fluoxastrobin, Flutriafol, Fluxapyroxad, Isopyrazam, Iprodione, Isotianil, Mancozeb, Maneb, Metconazole, Metominostrobin, Myclobutanil, Penthiopyrad, Picoxystrobin, Propiconazole, Propineb, Prothioconazole, Pyraclostrobin, Tebuconazole, Tetraconazole, Thiophanate-methyl, Trifloxystrobin; Sugarbeet Herbicides: Chloridazon, Desmedipham, Ethofumesate, Phenmedipham, Triallate, Clopyralid, Fluazifop, Lenacil, Metamitron, Quinmerac, Cycloxydim, Triflusulfuron, Tepraloxydim, Quizalofop; Sugarbeet Insecticides: Imidacloprid, Clothianidin, Thiamethoxam, Thiacloprid, Acetamiprid, Dinetofuran, Deltamethrin, β-Cyfluthrin, gamma/lambda Cyhalothrin, 4-[[(6-Chlorpyridin-3-yl)methyl](2,2-difluorethyl)amino]furan-2(5H)-on, Tefluthrin, Rynaxypyr, Cyaxypyr, Fipronil, Carbofuran; Canola Herbicides: Clopyralid, Diclofop, Fluazifop, Glufosinate, Glyphosate, Metazachlor, Trifluralin Ethametsulfuron, Quinmerac, Quizalofop, Clethodim, Tepraloxydim; Canola Fungicides: Azoxystrobin, Bixafen, Boscalid, Carbendazim, Cyproconazole, Difenoconazole, Dimoxystrobin, Epoxiconazole, Fluazinam, Fluopyram, Fluoxastrobin, Flusilazole, Fluxapyroxad, Iprodione, Isopyrazam, Mepiquat-chloride, Metconazole, Metominostrobin, Paclobutrazole, Penthiopyrad., Picoxystrobin, Prochloraz, Prothioconazole, Pyraclostrobin, Tebuconazole, Thiophanate-methyl, Trifloxystrobin, Vinclozolin; Canola Insecticides: Carbofuran, Thiacloprid, Deltamethrin, Imidacloprid, Clothianidin, Thiamethoxam, Acetamiprid, Dinetofuran, β-Cyfluthrin, gamma and lambda Cyhalothrin, tau-Fluvaleriate, Ethiprole, Spinosad, Spinotoram, Flubendiamide, Rynaxypyr, Cyazypyr, 4-[[(6-Chlorpyridin-3-yl)methyl](2,2-difluorethyl)amino]furan-2(5H)-on.

The following examples are offered by way of illustration and not by way of limitation.

### EXPERIMENTAL EXAMPLES

### Example 1. Expression and activity of Axmi184 in Zea mays

The Axmil84 gene (SEQ ID NO:1) was isolated from *Bacillus thuringiensis* strain ATX14775, and is described in U.S. Patent Publication 20100004176. The AXMI184 amino acid sequence (SEQ ID NO:3) has 93% sequence identity to Vip3Af1 (GENBANK Accession No. CAI43275). Vip3A proteins have been shown to be toxic to a taxonomically diverse group of Lepidoptera (Estruch et al. (1996) Proc Natl Acad Sci USA 93:5389-5394). There has been no evidence that any gene having homology to the VIP class of toxins is active against coleopteran pests.

Transgenic maize plants transformed with an optimized version of Axmi184 (optAxmi184, set forth herein as SEQ ID NO:2) were obtained by *Agrobacterium*-mediated transformation. Plants shown by PCR analysis to contain the appropriate Axmil84 construct were selected, and transferred to root trainer containers.

To plants containing optAxmi184 were transplanted to root-trainer containers and propagated for approximately three weeks. Individual plants were then each infested with ∼125 non-diapausing western corn rootworm (*Diabrotica virgifera*) eggs. Plants were analyzed for expression of AXMI184 protein by Western blot analysis using an anti-AXMI184 antibody. Plants expressing detectable amounts of AXMI184 were selected for analysis. After fifteen days, the amount of root damage in each plant was assessed using the Node Injury Scale (CRWNIS) (Oleson et al. 2005. J. Econ Entomol. 98(1): 1-8), where 0.00 is no injury and 3.00 is all roots pruned to within 1.5 inches of the stalk on three entire nodes. Figure 1 shows that AXMI184 resulted in lower root damage than control plants infested in the same manner.

### Example 2. Field trial results

T1 plants expressing optAxmi184 were tested at two locations in Iowa. Each experimental unit consisted of a one-row plot of maize measuring approximately 15 feet. Plots were arranged using a randomized complete block design with four replications. Efficacy against western corn rootworm was determined by evaluating up to eight maize roots per plot. Inbreds B110 and B116, a CRW-susceptible commercial hybrid, and two CRW control standards: B110 with a standard granular insecticide (FORCE® 3G), and B110 with an insecticidal seed treatment (PONCHO® 1250) were used as controls.

Fields were prepared for this study using cultural practices similar to those used in commercial maize fields in Iowa. Plants not containing transgenic events for CRW control were identified and excluded from the study using an application of 16 oz/A of glyphosate when plants were at the V1 leaf stage. Five to ten days later, the number of positive and negative plants in each plot was recorded. Negative plants were then removed and the remaining positive plants were thinned to 8 plants per row. All plants were then manually infested with non-diapausing western corn rootworm eggs. The eggs were suspended in a dilute agar solution and calibrated to deliver 750 eggs per plant for all plots.

Roots were dug, washed and evaluated for CRW injury after a period of larval feeding. Observations of larval development on unprotected maize (B110) were used to determine optimal timing for this evaluation. Corn rootworm injury was characterized using the CRWNIS. The results are shown in Table 1.

**Table 1**

| Plant | Number of plants | Average CRWNIS | Std Error |
|---|---|---|---|
| B110 | 58 | 1.53 | 0.075 |
| B110 + Force | 30 | 0.30 | 0.105 |
| B110 + Poncho | 31 | 0.08 | 0.103 |
| B116 | 56 | 1.52 | 0.077 |
| Negative hybrid | 32 | 0.83 | 0.101 |
| optAxmi184 | 175 | 0.12 | 0.043 |

### Example 3. Vectoring of Genes for Plant Expression

The coding regions of the invention are connected with appropriate promoter and terminator sequences for expression in plants. Such sequences are well known in the art and may include the rice actin promoter or maize ubiquitin promoter for expression in monocots, the *Arabidopsis* UBQ3 promoter or CaMV 35S promoter for expression in dicots, and the nos or PinII terminators. Techniques for producing and confirming promoter - gene - terminator constructs also are well known in the art.

In one aspect of the invention, synthetic DNA sequences are designed and generated. These synthetic sequences have altered nucleotide sequence relative to the parent sequence, but encode proteins that are essentially identical to the parent sequence.

In another aspect of the invention, modified versions of the synthetic genes are designed such that the resulting peptide is targeted to a plant organelle, such as the endoplasmic reticulum or the apoplast. Peptide sequences known to result in targeting of fusion proteins to plant organelles are known in the art. For example, the N-terminal region of the acid phosphatase gene from the White Lupin *Lupinus albus* (GENBANK® ID GI:14276838, Miller et al. (2001) Plant Physiology 127: 594-606) is known in the art to result in endoplasmic reticulum targeting of heterologous proteins. If the resulting fusion protein also contains an endoplasmic reticulum retention sequence comprising the peptide N-terminus-lysine-aspartic acid-glutamic acid-leucine (i.e., the "KDEL" motif, SEQ ID NO:4) at the C-terminus, the fusion protein will be targeted to the endoplasmic reticulum. If the fusion protein lacks an endoplasmic reticulum targeting sequence at the C-terminus, the protein will be targeted to the endoplasmic reticulum, but will ultimately be sequestered in the apoplast.

Thus, this gene encodes a fusion protein that contains the N-terminal thirty-one amino acids of the acid phosphatase gene from the White Lupin *Lupinus albus* (GENBANK® ID GI:14276838, Miller *et al.,* 2001, *supra*) fused to the N-terminus of the amino acid sequence of the invention, as well as the KDEL (SEQ ID NO:4) sequence at the C-terminus. Thus, the resulting protein is predicted to be targeted the plant endoplasmic reticulum upon expression in a plant cell.

The plant expression cassettes described above are combined with an appropriate plant selectable marker to aid in the selection of transformed cells and tissues, and ligated into plant transformation vectors. These may include binary vectors from *Agrobacterium-*mediated transformation or simple plasmid vectors for aerosol or biolistic transformation.

### Example 4. Transformation of genes of the invention in Plant Cells by Agrobacterium-Mediated Transformation

Ears are best collected 8-12 days after pollination. Embryos are isolated from the ears, and those embryos 0.8-1.5 mm in size are preferred for use in transformation. Embryos are plated scutellum side-up on a suitable incubation media, and incubated overnight at 25°C in the dark. However, it is not necessary *per se* to incubate the embryos overnight. Embryos are contacted with an *Agrobacterium* strain containing the appropriate vectors for Ti plasmid mediated transfer for about 5-10 min, and then plated onto co-cultivation media for about 3 days (22°C in the dark). After co-cultivation, explants are transferred to recovery period media for 5-10 days (at 25°C in the dark). Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated as known in the art.

All publications and patent applications mentioned in the specification are indicative of the level of skill of those skilled in the art to which this invention pertains.

Although the foregoing invention has been described in some detail by way of illustration and example for purposes of clarity of understanding, it will be obvious that certain changes and modifications may be practiced within the scope of the appended claims.

## Claims

1. A method for killing or controlling a corn rootworm pest population comprising contacting said population with a pesticidally-effective amount of a polypeptide comprising an amino acid sequence having at least 95% sequence identity to the amino acid sequence of SEQ ID NO:3, wherein the polypeptide has pesticidal activity against a corn rootworm pest.

2. A method for protecting a corn plant from a corn rootworm pest, comprising expressing in a corn plant or cell thereof a nucleotide sequence operably linked to a promoter capable of directing expression of the nucleotide sequence in a plant cell, wherein said nucleotide sequence is selected from the group consisting of:
a) the nucleotide sequence set forth in SEQ ID NO: 1 or 2; and
b) a nucleotide sequence that encodes a polypeptide comprising an amino acid sequence having at least 95% sequence identity to the amino acid sequence of SEQ ID NO:3, wherein said polypeptide has pesticidal activity against a corn rootworm pest.

3. A method for increasing yield in a corn plant comprising growing in a field a corn plant of or a seed thereof having stably incorporated into its genome a DNA construct comprising a nucleotide sequence operably linked to a promoter capable of directing expression of the nucleotide sequence in a plant cell, wherein said nucleotide sequence is selected from the group consisting of:
a) the nucleotide sequence set forth in SEQ ID NO: 1 or 2; and
b) a nucleotide sequence that encodes a polypeptide comprising an amino acid sequence having at least 95% sequence identity to the amino acid sequence of SEQ ID NO:3, wherein said polypeptide has pesticidal activity against a corn rootworm pest; wherein said field is infested with a corn rootworm pest.

4. The method of claims 1-3, wherein said pest is a Western corn rootworm pest.

5. The method of claims 2 or 3, wherein said plant further comprises one or more nucleotide sequences encoding one or more additional insect toxins.

6. The method of any one of the preceding claims wherein the polypeptide comprises an amino acid sequence having at least 98% sequence identity to the amino acid sequence of SEQ ID NO: 3.

7. Use of the plant of claims 2 or 3 for the control of a corn rootworm pest.

## Patentansprüche

1. Verfahren zum Abtöten oder Einschränken einer Maiswurzelbohrer-Schädlingspopulation, umfassend ein In-Kontakt-bringen der Population mit einer pestizidwirksamen Menge eines Polypeptids, das eine Aminosäuresequenz umfasst, die mindestens 95 % Sequenzidentität mit der Aminosäuresequenz von SEQ ID NR: 3 aufweist, wobei das Polypeptid eine pestizide Aktivität gegen einen Maiswurzelbohrer-Schädling aufweist.

2. Verfahren zum Schützen einer Maispflanze vor einem Maiswurzelbohrer-Schädling, umfassend ein Exprimieren einer Nukleotidsequenz in einer Maispflanze oder Zelle davon, die funktionsfähig mit einem Promotor verbunden ist, der fähig ist, eine Expression der Nukleotidsequenz in einer Pflanzenzelle zu steuern, wobei die Nukleotidsequenz ausgewählt ist aus der Gruppe bestehend aus:
a) der Nukleotidsequenz gemäß SEQ ID NR: 1 oder 2; und
b) einer Nukleotidsequenz, die ein Polypeptid kodiert, das eine Aminosäuresequenz umfasst, die mindestens 95 % Sequenzidentität mit der Aminosäuresequenz von SEQ ID NR: 3 aufweist, wobei das Polypeptid eine pestizide Aktivität gegen einen Maiswurzelbohrer-Schädling aufweist.

3. Verfahren zum Erhöhen eines Ertrags einer Maispflanze, umfassend ein Anbauen einer Maispflanze oder eines Samens davon in einem Feld, die ein stabil in ihr Genom integriertes DNA-Konstrukt aufweist, das eine Nukleotidsequenz umfasst, die funktionsfähig mit einem Promoter verbunden ist, der fähig ist, eine Expression der Nukleotidsequenz in einer Pflanzenzelle zu steuern, wobei die Nukleotidsequenz ausgewählt ist aus der Gruppe bestehend aus:
a) der Nukleotidsequenz gemäß SEQ ID NR: 1 oder 2; und
b) einer Nukleotidsequenz, die ein Polypeptid kodiert, das eine Aminosäuresequenz umfasst, die mindestens 95 % Sequenzidentität mit der Aminosäuresequenz von SEQ ID NR: 3 aufweist, wobei das Polypeptid eine pestizide Aktivität gegen einen Maiswurzelbohrer-Schädling aufweist; wobei das Feld von einem Maiswurzelbohrer-Schädling befallen ist.

4. Verfahren nach Ansprüchen 1 bis 3, wobei der Schädling ein Westlicher Maiswurzelbohrer-Schädling ist.

5. Verfahren nach Ansprüchen 2 oder 3, wobei die Pflanze ferner eine oder mehrere Nukleotidsequenzen umfasst, die ein oder mehrere zusätzliche Insektentoxine kodieren.

6. Verfahren nach einem der vorstehenden Ansprüche, wobei das Polypeptid eine Aminosäuresequenz aufweist, die mindestens 98 % Sequenzidentität mit der Aminosäuresequenz von SEQ ID NR: 3 aufweist.

7. Verwendung der Pflanze nach Ansprüchen 2 oder 3 zur Einschränkung eines Maiswurzelbohrer-Schädlings.

## Revendications

1. Procédé visant à tuer ou lutter contre une population nuisible de chrysomèles des racines du maïs comprenant la mise en contact de ladite population avec une quantité efficace du point de vue pesticide d'un polypeptide comprenant une séquence d'acides aminés présentant au moins 95 % d'identité de séquence à la séquence d'acides aminés de la SEQ ID n° : 3, le polypeptide présentant une activité pesticide contre une chrysomèle des racines du maïs.

2. Procédé de protection d'un plant de maïs contre une chrysomèle des racines du maïs, comprenant l'expression dans un plant de maïs ou une cellule de celui-ci d'une séquence nucléotidique liée de manière fonctionnelle à un promoteur capable de diriger l'expression de la séquence nucléotidique dans une cellule végétale, ladite séquence nucléotidique étant choisie dans le groupe constitué par :
a) la séquence nucléotidique indiquée dans la SEQ ID n° : 1 ou 2 ; et
b) une séquence nucléotidique qui code pour un polypeptide comprenant une séquence d'acides aminés présentant au moins 95 % d'identité de séquence à la séquence d'acides aminés de la SEQ ID n° : 3, ledit polypeptide présentant une activité pesticide contre une chrysomèle des racines du maïs.

3. Procédé destiné à augmenter le rendement dans un plant de maïs comprenant la croissance dans un champ d'un plant de maïs ou d'une semence de celui-ci comportant incorporé de manière stable dans son génome une structure d'ADN comprenant une séquence nucléotidique liée de manière fonctionnelle à un promoteur capable de diriger l'expression de la séquence nucléotidique dans une cellule végétale, ladite séquence nucléotidique étant choisie dans le groupe constitué par :
a) la séquence nucléotidique indiquée dans la SEQ ID n° : 1 ou 2 ; et
b) une séquence nucléotidique qui code pour un polypeptide comprenant une séquence d'acides aminés présentant au moins 95 % d'identité de séquence à la séquence d'acides aminés de la SEQ ID n° : 3, ledit polypeptide présentant une activité pesticide contre une chrysomèle des racines du maïs ; ledit champ étant infesté par une chrysomèle des racines du maïs.

4. Procédé selon les revendications 1 à 3, dans lequel ledit ravageur est une chrysomèle des racines du maïs occidentale.

5. Procédé selon les revendications 2 ou 3, dans lequel le plant comprend en outre au moins une séquence nucléotidique codant pour au moins une toxine d'insecte supplémentaire.

6. Procédé selon l'une quelconque des revendications précédentes, dans lequel le polypeptide comprend une séquence d'acides aminés présentant au moins 98 % d'identité de séquence à la séquence d'acides aminés de la SEQ ID n° : 3.

7. Utilisation du plant selon les revendications 2 ou 3 destinée à la lutte contre une chrysomèle des racines du maïs.
